# EUROPEAN PATENT APPLICATION

(11) **EP 4 144 258 A1**
(43) Date of publication of application: **08.03.2023**
(21) Application number: 21194595.1
(22) Date of filing: 02.09.2021
(51) Int. Cl.: A45F 3/22, A61G 11/00, A47D 7/04, A47D 9/02, A47D 13/02, A61B 5/00

(54) **HAMMOCK**

(71) Applicant: Kluba Medical GmbH, 40223 Düsseldorf (DE)
(72) Inventor: ALTHOFF, Björna, 40223 Düsseldorf (DE)
(74) Representative: König Szynka Tilmann von Renesse Patentanwälte Partnerschaft mbB Düsseldorf

(57) **Abstract**

The present invention relates to a lying and transport device for an infant comprising a hammock and methods of monitoring the well-being and/or vital signals of an infant.

## Description

### Field of the invention

The present invention relates to a lying and transport device for an infant comprising a hammock and methods of monitoring the well-being and/or vital signals of an infant.

### Background of the invention

Premature (preterm) infants experience a variety of non-physiological stimuli from light, noise, touch, and pain in the neonatal intensive care unit compared with fetuses in the uterus of the same developmental stage. At the same time, they experience deprivation of physiological stimuli such as intrauterine acoustics or movement. There are numerous studies that examine how the development and outcome of preterm infants can be improved when they are provided with a developmentally supportive environment. The present invention in particular aims at the improvement of motoric function and vestibular stimulation:

Premature infants possess all the anatomical basics for movement. However, they have not been able to further develop these skills under intrauterine conditions through self-motion as well as movement by the maternal body. In addition to a developmental deficit, premature infants exhibit generalized muscular hypotonia based on immaturity. This, along with gravity counteracting many movements, makes it difficult for premature babies to make movements on their own.

It is known that vestibular stimulation through positioning on hammock or waterbed promotes the development and outcome of premature infants (Neal M (1968) Vestibular stimulation and developmental behaviour of the small premature infant. Nursing Research Report 3:1-5). Furthermore, it can improve sleep and oxygen saturation and reduce heart- and respiratory rat (Jesus VR, Oliveira PMN, Azevedo VMGO. Effects of hammock positioning in behavioural status, vital signs, and pain in preterms: a case series study. Braz J Phys Ther. 2018 Jul-Aug;22(4):304-309, Ribas CG, Andreazza MG, Neves VC, Valderramas S. Effectiveness of Hammock Positioning in Reducing Pain and Improving Sleep-Wakefulness State in Preterm Infants. Respir Care 2019;64(4):384-389.). Other studies found a higher score of neuromuscular maturity and also a reduction in heart rate and respiratory rate over a 10-day study interval with 3-hour hammock positioning per day (Keller A, Arbel N, Merlob P, Davidson S. Neurobehavioral and autonomic effects of hammock positioning in infants with very low birth weight. Pediatr Phys Ther 2003; 15(1):3-7).

Positioning change is associated with stress and painful expression of the prematures (Ganguly A, Bhadesia PJ, Phatak AG, Nimbalkar AS, Nimbalkar SM. Pain profile of premature infants during routine procedures in neonatal intensive care: An observational study. J Family Med Prim Care. 2020;9(3):1517-1521. Published 2020 Mar 26. doi:10.4103/jfmpc.jfmpc_1033_19). In this context, interventions to facilitate a calm behavioural state and motor organization in the infant, addressing positioning and handling of the infant, as well as movement therapy are needed.

The skin-to-skin contact between infant and parent called "kangaroo care" (KC) has been shown to reduce mortality, severe illness, infection and length of hospital stay in developing countries for low-birthweight infants. "KC is also beneficial for preterm infants in high-income countries. Cardiorespiratory and temperature stability, sleep organization and duration of quiet sleep, neurodevelopmental outcomes, breastfeeding and modulation of pain responses appear to be improved for preterm infants who have received KC during their hospital stay" (Jefferies AL; Canadian Paediatric Society, Fetus and Newborn Committee. Kangaroo care for the preterm infant and family. Paediatr Child Health. 2012 Mar;17(3):141-6. doi: 10.1093/pch/17.3.141. PMID: 23449885; PMCID: PMC3287094.).

In order to provide the environment for infants in NICU, it is known to use hammocks in new born incubators. NL 9 100 645 A discloses a device for use in an incubator, in which a hammock is suspended from the four corners of a frame, particularly by grommets in the hammock from hooks on the frame. This hammock does not allow a safe detachment and mounting of the hammock with the infant lying therein.

WO2015070969A1 discloses a thermotherapy device which includes a hammock that can be pulled and/or pivoted out of the incubator chamber. However, this does not allow a removal of the infant lying on the textile outside the rack or a flexible use in other incubator types.

DE 200 06 758 U1 discloses an incubator with a hammock comprising a lying area connected by a plurality of bands to a mounting means, particularly a grommet, at each transverse side. The hammock can then be suspended from hooks attached to the incubator. The expired patent DE20006758U1 does not allow an easy and safe removal of the hammock together with the infant.

### Summary of the Invention

It is the objective of the invention to overcome at least one of the drawbacks of the prior art.

In particular the invention aims at providing for a preterm infant an environment that promotes social and/or physiological development by reducing stress by easy and safe translocating or less manipulation of the infant in the NICU. This problem is overcome by the provision of a device according to claim 1. Particular advantageous embodiments, such as e.g. suitable means swinging the hammock, are subject matter of the dependent claims and/or further independent claims.

The invention relates to a device for lying and translocating of an infant comprising a hammock with a lying area and at least one fastening means and at least one mounting means, said mounting means is designed to spread the lying area at least partially while translocating the infant. The mounting means is reversibly mountable and detachable from said fastening means in a two-handed operation.

The fastening means preferably is located at the head end as well as at the foot end of the hammock. It can be formed in one piece or in two or more pieces. The fastening means serves to fasten the hammock on a support frame. The fastening means can be attached to the support frame either centrally or at more than one position.

It is alike with the mounting means. It can be formed as one-piece or in two or more pieces and is located preferably at the head end and the foot end of the hammock.

This device allows to remove (mounting and detaching) the hammock together with the infant from the fastening means and to translocate it. This is possible as a one-person only operation. Thereby the infant can be translocated to other places for medical care or, in particular to a reference person (e.g mother or father). At these places, the infant can even remain in the hammock. Accordingly, the device according to the invention reduces the need for manipulation, and thus stress, for the infant.

Due to the mounting means spreading the lying area of the hammock at least partially during the translocation, the transfer of the infant is safe and convenient. The ease of transfer enables a more frequent but rather unstressful translocation; thereby allowing a better social and physiological development of the infant. Thus, it is one particular important advantage of the invention to simplify the kangarooing of the infant during intensive care.

The safety of translocating the infant is further improved when the hammock comprises a cover element that, preferably, at least partially embraces the infant. This cover element preferably serves twofold, as on the one hand it is a fastening mean to secure the infant on the hammock. On the other hand, it can be used for "swaddling" (synonyms are "wrapping" or "pucking") the infant. For the swaddling function it is particularly advantageous when the cover element is large enough to embrace the entire body of the infant. The cover element preferably is connected with the hammock. The connection preferably is reversible. The cover element can be designed with name letters or symbols by the parents with textile painters, which are compatible with preterm skin.

In yet another aspect of the invention the device comprises actuating means. These serve to set the hammock into motion. Preferably the actuating means are connected with the fastening means or are part thereof. The motion preferably is swinging around a central axis. Preferably, the mounting means spread the hammock at least partially in the transverse direction to this central axis. This allows a safe motion; and, furthermore, a safe translocation when the hammock together with the infant is translocated.

The device according to the invention can be a stand-alone device. In one preferred embodiment it is, however, part of an incubator. This is typically used in an NICU. In this embodiment, the fastening means of the hammock can either be connected to a support frame that is comprised in the incubator, or, in an alternative embodiment, the fastening means are attached to the incubator. In this latter embodiment a support frame is not required. If a support frame is used, preferable, it can be adjustable in its dimensions, in particular in height. This allows an easy fit into incubators of various sizes.

In one particular aspect the invention relates to a hammock comprising at least one sensor for the monitoring of vital signals of the infant. The sensor is attached to, more preferred integrated into the hammock, e.g. in form of a smart textiles (so called "textrodes"). It is most preferred that the sensor (in particular the textrode) is reverseble integrated into the lying area of the hammock. In a particularly preferred embodiment, the sensor is located in the lying area, where the buttock or the back of the infant are expected when the infant is in the hammock. Due to the weight of the infant, thereby, a suitable connection between the sensor on the infant's body is ensured.

In yet another embodiment of the invention the electrode, in particular the textrode, can be integrated into the cover element. Also, a combination is possible, i.e. electrodes attached to or integrated in the hammock (as above) with electrodes in the cover element.

In one particular embodiment of the invention, thus a method is provided for the monitoring of the vital signs of an infant. This method comprising the steps of placing the infant into a hammock with a lying area comprising at least one sensor, connecting the sensor with the infant, in particular on its buttock or back and recording and/or surveying the signals.

Particular aspects and embodiments of the invention are described in detail below.

### Detailed Description of the Invention

The present invention is illustrated by way of example by the following particular aspects and preferred embodiments thereof. Preferably, one or more aspects are combined; most preferred all aspects are combined.

The hammock preferably is a textile. This textile can be replaceable and reusable to enable hygienic reprocessing. The material properties therefore also preferably allow it to be washed at a minimum of 60 °C.

The design of the hammock can include upright sides as a lateral textile boundary of the rectangular hammock. These upright sides as well as small padding or cushions, which can be used as positioning devices next to the infant's sides or under its legs, serve as elements for more comfort, more support and/or prevention of slipping out. In addition, embracing means can be connected (e.g. sewn or detachable connected with push buttons), which can serve swaddling (wrapping, pucking), similar to intrauterine conditions and a better retention of the body temperature. All textiles can be supplemented by the integration of active or sensory textiles such as heating elements or electrode for recording vital parameters.

The hammock (in particular in the form of a textile) can also have receptacles and devices that can accommodate other therapeutic and sensory elements, e.g. non-textile elements. All textile elements (e.g. such as the cover element, upholstery, side surfaces, textrodes) can be implemented either permanently with the lying area or as reversible modules for it. The hammock can be made of washable textiles or single use materials.

At the head- and foot-end, the lying area can have interfaces that allow the textile to be attachable to the mounting means, but also to be detached from it again. Sewn-in loops or tunnels as well as eyelets or hooks are possible.

These elements can also be used on a standard mattress as a wrapping blanket. It offers a textile part for the back on which the infant can be laid in the supine position as well as two lateral textile wrapping elements that can be brought together over the infant's stomach. The embracing means can have a size-adaptable fastening mechanism, such as a pull-through textile opening and a knot behind or with it, or buttons, Velcro fasteners, eyelets and straps.

The above-mentioned integration of sensors into the lying area of the hammock or into the cover element can be combined with any type of mattress or in any type of mounting or fastening means. Hence, their use is not limited to the device as of the present claim 1. Both textile sensors and sensors integrated in the textile are known in the art. Preferred is a wrinkle-free fit of the textile to the infant's back enabling good contact pressure. This allows a good derivation of the measured signals.

For example, textrodes made of woven or moos sticked silver or gold yarns can be integrated, which can derive an ECG to assess the ECG curve, heart rate and respiratory rate. Textrodes like non-textile sensors can be reversibly connected to textile conductors that can be fitted to the lying area. These sensors can include photoplethysmography, temperature sensors, humidity sensors, ballistocardiography sensor, accelerometers or capnography sensors. They have to be designed to "withstand" corrosion in high oxygen concentration at temperatures/humidity common in an incubator. For active heating, copper elements can be sewn in, for example, which must have their own thermoregulation unit to prevent excessive heating, for example.

The hammock can be attached by its adapters at the foot and head end respectively to a mounting means that has a counterpart to this adapter. This can be, for example, an attached or removable pole in an elongated recess that can accommodate sewn-in loops or tunnels. A safety mechanism is used to prevent the textile from slipping out of the mounting means. The mounting means can have an ergonomic part where the hands of the person removing it can grip firmly. In addition, the mounting means has components with which it can be fixed to a frame or moved through an intermediate element of a frame. These components can be, for example, extensions of the mounting means, hooks or eyelets. For an addition of smart textiles, the mounting means can also be used as an interface from textile conductors to further electronics: It can include a housing or a mounting option for the interface with cable connections, circuit boards, power supply, battery and data transfer. The construction allows the mounting means to be attached to an external structure with fastening means. This fastening means can be a single, movable component or an integral part of a scaffolding element. It can have a U-shape, a box, hooks or eyelets to receive the counterparts of the mounting means. It is designed in such a way that at the same time a secure placement of the mounting means but also an easy, two-handed removal and inserting is possible. The fastening means can have a greater distance than the length of the head or foot end of the lying area textile if the components of the mounting means overlap the corners of the textile. The way of the connection or integration of the fastening means to the incubator or stable structure (like the frame) facilitates a movement in at least two dimensions. This connection can be realised by a mounting to the device which facilitates height adjustability.

In a preferred embodiment, the present invention offers the possibility that a premature infant together with its surrounding textile and the attached sensor technology can be removed from the incubator easily and with reduced stress for the infant. It is a sort of a "textile tray", and enables the easy translocation in the arms of a person or a medical device, such as e.g. an X-ray plate.

In one preferred embodiment the fastening means are opened upwards in a U-shape and the mounting means can be inserted into the U-shaped holder from above. Hence the operator can remove the hammock by grasping the mounting means with an easy vertical lift. Depending on the frame to which the fastening means are attached, a simple parallel movement of the hands out of the lateral open sides of the incubator can be sufficient. If there are elements of the frame inhibiting this direct movement, there is a lifting and horizontal rotation necessary previous to the movement out of the sides. Another alternative is a pronation of the hands holding the mounting means to shorten the length of the "textile tray" and thus a possibility to avoid barriers by the frame.

The fastening elements can be attached to a device which facilitates height adjustability. This device can be the incubator itself or a support frame with holes or hooks at different heights. Otherwise the height adjustability is given by a moveable element of the support frame which can be fixed at different heights on parts of the support frame. Alternatively, the height adjustability can be realised via an attachment of the fastening means to linking elements like rods, which are variable in their length by knots or similar techniques. The height adjustability enables a laying down of the lying area to the ground as well as heights which enable the rocking of the hammock or a placement of other devices such as waterbeds under the lying area.

The fastening element preferably is set into motion by actuating means. This movement is performed preferably in an alternating movement around the central axis of the fastening elements to induce swinging of the hammock.

The actuating means can be placed to the head- and foot-end for synchronous movement and can offer a variability of movements: from child induced passive movement only to multidimensional movements adjustable in amplitude and frequency, inhibiting movements that could be a danger for the child lying in the hammock.

The actuating means can include means for a programmed movement, i.e. a sequence of motions over time and/or remote control. The power supply can be given by cable connection to the grid and/or a rechargeable battery. The actuating means can be integrated into the height adjustability module and is designed to withstand the conditions such as oxygen concentration, temperatures and humidity common in an incubator.

The above-mentioned elements can be used for an installation connected to elements of an incubator or an installation in a support frame. The support frame preferably comprises a flat ground and collapsible or removable one to two upright pieces (poles), preferably at the head- and foot-end located in the position. This is advantageous for the moveable connection of one or two fastening means, for example in the middle of the respective side. Its shape, size and collapsibility or installation of the upright pieces facilitates the lateral inserting into common incubators.

The collapsibility can be realised by a hinge or pivot installed between the upright pieces and the ground. The support frame preferably has a mechanism which inhibits a bending during the installation when the hammock is mounted.

The upright pieces can include holes or hooks for an attachment in different heights or offer the opportunity for a connection with a height adjustability device and/or the connection with actuating means.

The used materials such as plastics or metals preferably disinfectable; e.g. with disinfection liquids or high temperatures. Preferably, they allow X-ray examinations through the installed support frame.

The materials and way of installation is preferably chosen and designed to "withstand" corrosion in high oxygen concentration at temperatures/humidity common in an incubator. For empowering parents to create a lovely surrounding for their infant during its first days of life in a medical and technical environment, the support frame has mounting elements for decorative means such as buntings.

### Brief Description of the Figures

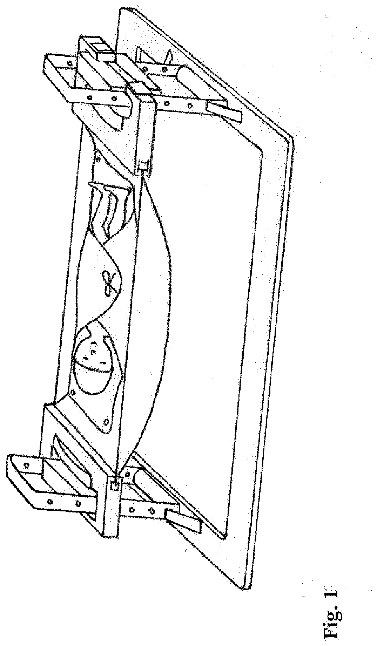

**Fig. 1** shows an infant lying in a device according to the invention. Detailed description aspects of the device are given in the following figures.

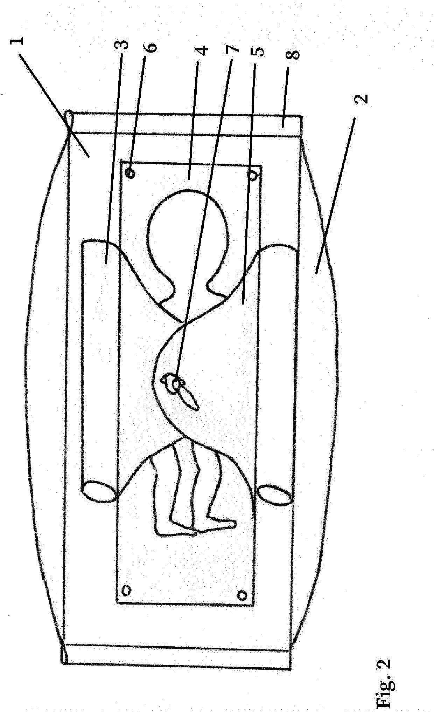

Fig. 2 shows an infant lying in the hammock aspect of a device according to the invention. The structure includes a textile as the central lying area (1), which has the function of a hammock. This textile is replaceable and reusable to enable hygienic reprocessing. The material properties therefore also allow it to be washed at a minimum of 60 °C. The hammock design includes limiting upright sides (2) beyond a rectangular area as well as padding (3) for support and prevention of slipping out. In addition, embracing means (4) with lateral textile wrapping elements/cover blankets (5) are attached with push buttons (6), which can take over the function of a comfortable embracing element (pucking) similar to intrauterine conditions and a better retention of the body temperature. The embracing means (4) can also be used as a standalone on a standard mattress. It offers a part on which the infant can be laid in the supine position as well as two lateral textile wrapping elements that is brought together over the infant's stomach. The insertion elements have a size-adaptable fastening mechanism by a pull-through textile opening and a knot (7) behind it. At the head and foot end, the lying area has interfaces in form of sewn-in loops (8) that attach the textile to a mounting means (15), but are also detached from it again.

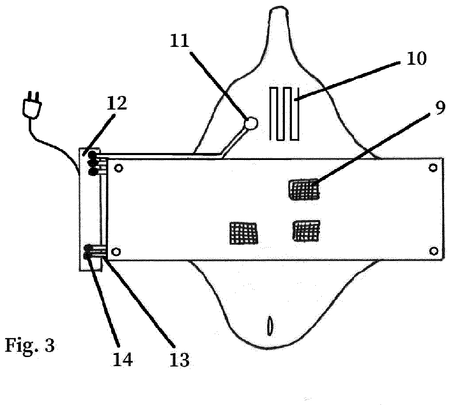

Fig. 3 shows the embracing means (4), which are supplemented by the integration of smart textile textrodes made with silver yarn and hydrophilic fibres for recording the vital parameters ECG, respiratory frequency and pulse rate (9). For the measurement of the body temperature, textile temperature sensors (10) are integrated as well. The textile has a receptacle that accommodates a non-textile photoplethysmography sensor (11) or a non-textile temperature sensor. For power supply and data transfer an additional interface (12) is connected to the sensor elements (9, 10, 11) with textile conductor paths (13) and conducting buttons (14). The smart textile embracing means (4) can also be used as a standalone during kangaroo care or on a standard mattress.

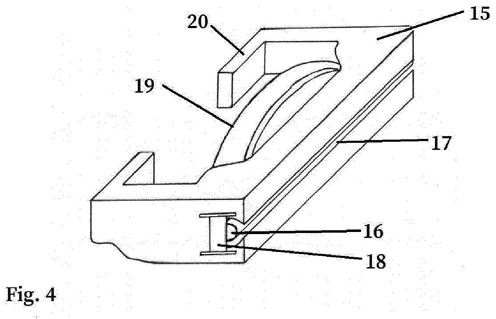

**Fig. 4** shows the mounting means (15), which offer the opportunity to use the hammock as a "textile tray" and/or to attach it to fastening means (21). The textile sewn-in tunnels loops at the foot and head end have poles (16) as counterparts in this adapter. These rods are attached in an elongated recess (17) that can accommodate the textile sewn-in loops (8). A safety mechanism (18) is used to prevent the textile from slipping out of the mounting means. The mounting means has an ergonomic part (19) where the hands of the person removing it can grip firmly. In addition, the mounting means has extensions of the mounting means (handle extensions) (20) as components for fixing it to a fastening element means (21).

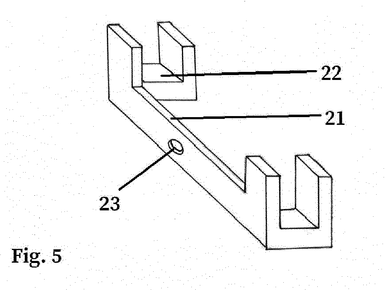

**Fig. 5** shows the fastening means (21), by which the mounting means (15) are attachable to an external structure. These fastening means are movable components and have U-shape hooks (22) to receive the handle extensions (20). It is designed in such a way that at the same time a secure placement of the mounting means but also an easy, two-handed removal and inserting is possible. The way of the connection or integration of the fastening means (21) to the incubator or stable structure (like the frame) facilitates a movement in at least two dimensions around a central axis (23).

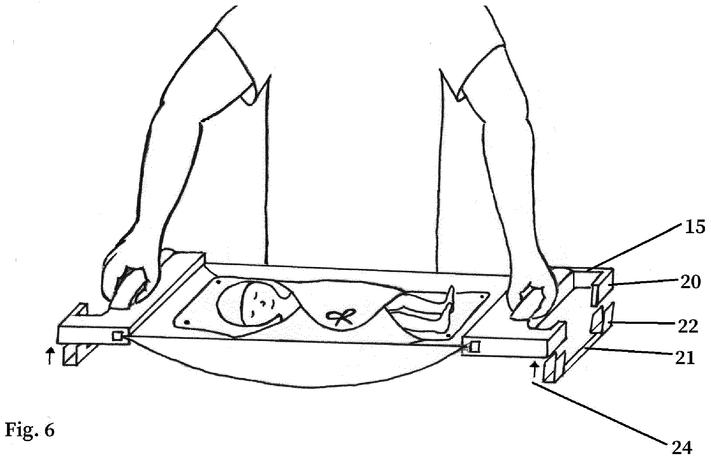

**Fig. 6** shows the one person-two handed operation of removing an infant including the hammock from a device according to the invention. By lifting the mounting means (15) in a vertical direction (24), the handle extensions (22) are detached from the hooks (22) of the fastening means (22).

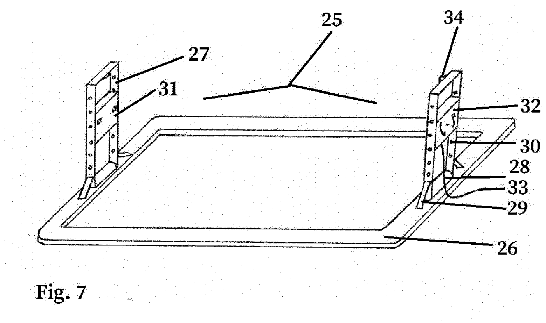

**Fig. 7** shows the support frame (25), which consists of a flat ground (26) and two collapsible and removable upright pieces (poles) (27) located in the middle of the head- and foot-end. Its shape, size and collapsibility or installation of the upright pieces (27) facilitate the lateral insertion into common incubators. The collapsibility is realised by a hinge (28) installed between the upright pieces and the ground. Due to the possibility of collapsibility, the support frame has a mechanism (29) which inhibits any bending during the installation with a mounted hammock. The upright pieces have holes (30) for an attachment in different heights of a height adjustability device (31) which includes actuating means (32). The actuating means receive electricity via a cable connection (33) to an electrical outlet.

### Example

An example of the invention with preferred embodiments illustrates the invention, without limiting it, and is described as follows.

The device includes a textile as the central lying area (1), which has the function of a hammock. This textile is replaceable and reusable to enable hygienic reprocessing. The material properties therefore also allow it to be washed at a minimum of 60° Celsius. The hammock design includes limiting upright sides (2) beyond a rectangular area as well as padding (3) for support and prevention of slipping out. In addition, embracing means (4) with lateral textile wrapping elements/cover blankets (5) are attached with push buttons (6), which can take over the function of a comfortable confinement/embracing element (pucking) similar to intrauterine conditions and a better retention of the body temperature. The embracing element (4) can also be used as a standalone on a standard mattress. It offers a part on which the infant can be laid in the supine position as well as two lateral textile wrapping elements that can be brought together over the infant's stomach. The insertion elements have a size-adaptable fastening mechanism by a pull-through textile opening and a knot (7) behind it.

At the head and foot end, the lying area has interfaces in form of sewn-in loops (8) that allow the textile to be attached to a mounting means, but also to be detached from it again.

The embracing means (4) can be supplemented by the integration of smart textile textrodes made with silver yarn and hydrophilic fibres for recording the vital parameters ECG, respiratory frequency and pulse rate (9). For the measurement of the body temperature, textile temperature sensors (10) are integrated as well. The textile has a receptacle that can accommodate a non-textile photoplethysmography sensor (11) or a non-textile temperature sensor. For power supply and data transfer an additional interface (12) is connected to the sensor elements (9, 10, 11) with textile conductor paths (13) and conducting buttons (14).

The Smart textile embracing means can also be used as a standalone during kangaroo care or on a standard mattress.

The hammock (textile) is attached to mounting means (15) to offer the opportunity to use the hammock as a "textile tray" or to attach it to fastening means (21). The sewn-in loops at the foot and head end have poles (16) as counterparts in this adapter. These rods are attached in an elongated recess (17) that can accommodate the sewn-in loops (8). A safety mechanism (18) is used to prevent the textile from slipping out of the mounting means. The mounting means can have an ergonomic part (19) where the hands of the person removing it can grip firmly. In addition, the mounting means can have handle extensions (20) as components for fixing it to a fastening means (21).

The construction allows the mounting means (15) to be attachable to an external structure via fastening means (21). These fastening means can be movable components and can have U-shape hooks (22) to receive the handle extensions (20). It is preferably designed in such a way that at the same time a secure placement of the mounting means but also an easy, two-handed removal and inserting is possible.

The way of the connection or integration of the fastening means (21) to the incubator or stable structure (like the frame) facilitates a movement in at least two dimensions around a central axis (23).

By means of the elements (1) (8), (15) and (21), the present invention offers the possibility that a premature infant together with its surrounding textile and the attached sensor technology can be removed from the incubator easily and without great positioning stress (as described in the introduction), as if on a "textile tray", to place it, for example, in the arms of the parents or on an X-ray plate. The fastening means (21) can have U-shaped hooks (22), which the handle extensions (20) can be inserted into from above. A removal can be realised by grasping the ergonomic parts (19) of the mounting means (15) at the head and foot ends and detach them from the fastening means (21) with a vertical lift (24). Because of elements of the frame inhibiting a direct movement, there is a lifting and horizontal rotation necessary previous to the movement out of the sides. Another alternative is a pronation of the hands holding the mounting means to shorten the length of the "textile tray" and thus a possibility to avoid barriers by the frame. A simple parallel movement of the hands out of the lateral open sides of the incubator is sufficient to move the infant on the "textile tray" out of the incubator.

The above-mentioned elements can be used for an installation connected to elements of an incubator or an installation in a support frame (25). The support frame consists of a flat ground (26) and collapsible and/or removable one to two upright pieces (poles) (27) located in the middle of the head- and foot-end. Its shape, size and collapsibility or installation of the upright pieces (27) facilitates the lateral insertion into common incubators. A collapsibility is be realised by a hinge (28) installed between the upright pieces and the ground. Due to the possibility of collapsibility the support frame has a mechanism (29), which inhibits any bending during the installation with a mounted hammock.

The upright pieces have holes (30) for an attachment in different heights of a height adjustability device (31), which includes actuating means (32). The height adjustability enables a laying down of the lying area to the ground as well as heights which enable the swinging of the hammock or a placement of other devices such as waterbeds under the lying area.

The mounting means and the infant lying in the hammock can be moved by active movement of the fastening means (21) performed by the actuating means (32). This movement can be performed in an oscillating motion around the central axis (23) of the fastening means (21) to induce swinging of the hammock. The actuating means can be placed to the head- and foot-end for synchronous movement and can offer a variability of movements: from infant induced passive movement only to multidimensional movements adjustable in amplitude and frequency, inhibiting movements that could be a danger for the infant lying in the hammock. The actuating means (32) includes the opportunity to program movement sequences for a specific time and via remote control. The actuating means can receive electricity via a cable connection (33) to an electrical outlet. The actuating means can also be connected to a rechargeable battery.

For empowering parents to create a lovely surrounding for their infant during its first days of life in a medical and technical environment, the support frame has mounting elements (34) for decorative means such as buntings.

## Claims

1. Device for lying and translocating of an infant comprising a hammock with a lying area (1), at least one fastening means (21), preferably two fastening means, and at least one mounting means (15), preferably two mounting means, said mounting means (15) is designed to spread the lying area (1) at least partially while translocating the infant and said at least two mounting means (15) are reversibly mountable and detachable from said fastening means (21) in a two-handed operation.

2. Device according to claim 1, **characterised in that** the mounting means (15) are simultaneously reversibly mountable and detachable from the fastening means (21) by a one-person operation.

3. Device according to claim 1 or 2, **characterised in that** the fastening means are part of or attachable to a support frame (25).

4. Device according to one or more of the previous claims, **characterised in that** either the fastening means (21) and/or the support frame (25) are adjustable in height.

5. Device according to claim 3 or 4, **characterised in that** the support frame (25) is at least partially collapsible.

6. Device according to one or more of the previous claims, **characterised in that** the fastening means (21) and/or the support frame (25) are attachable to an incubator.

7. Device according to one or more of the previous claims, **characterised in that** the device comprises at least one cover element (5) for swaddling the infant.

8. Device according to claim 7, characterized that the cover element is reversibly connected to the hammock.

9. Device according to claims 7 or 8, characterized that the cover element (5) is part of an embracing means (4), which allows essentially embracing the entire body of the infant.

10. Device according to one or more of the previous claims, **characterised in that** it comprises at least one sensor for vital signs of the infant, preferably attached to or integrated in the lying area (1) of the hammock and/or the cover element (5) according to claims 7 to 9, in particular allowing contact to the infant's skin.

11. Device according to one or more of the previous claims, **characterized in that** it comprises actuating means (32), preferably attached to or integrated to the fastening means (21) and/or mounting means (15).

12. Device according to claim 11, **characterized in that** the actuating means (32) set the hammock into motion along the central axis (23).

13. Device according to claims 12, **characterized in that** the mounting means (15) spread the hammock at least partially traverse to the central axis (23) when the hammock is mounted.

14. Device according to any of the previous claims integrated into an incubator for newborns.

15. Method of monitoring vital signals of an infant comprising the steps of placing the infant into a hammock with a lying area (1) comprising at least one sensor (9, 10, 11), connecting the sensor with the infant, and recording and/or surveying the signals.
